# EUROPÄISCHE PATENTSCHRIFT

(11) **EP 0 380 001 B1**
(45) Veröffentlichungstag und Bekanntmachung des Hinweises auf die Patenterteilung: **06.04.1994**
(21) Anmeldenummer: 90101132.0
(22) Anmeldetag: 20.01.1990
(51) Int. Cl.: B01D 3/14, C07C 45/82

(54) **Verfahren zur destillativen Abtrennung geringer Mengen einer Mittelsiederfraktion aus einem Flüssigkeitsgemisch**
Method for the separation by distillation of small quantities of middle boiling fractions from a liquid-mixture
Procédé de séparation destillative de quantité minime d'une fraction d'éballonnement moyenne d'une mélange de liquide

(30) Priorität: 25.01.1989 DE 3902006
(43) Veröffentlichungstag der Anmeldung: 01.08.1990
(73) Patentinhaber: BASF Aktiengesellschaft, 67063 Ludwigshafen (DE)
(72) Erfinder: Kaibel, Gerd, Dr., D-6840 Lampertheim (DE); Schloemer, Karl, Dr., D-6700 Ludwigshafen (DE); Mayer, Hans-Horst, D-6720 Speyer (DE)

(56) Entgegenhaltungen:
- US-A- 3 058 893
- US-A- 4 744 869
- CHEMIE-INGENIEUR-TECHNIK, Band 61, Nr. 2, Februar 1989, Seiten 104-112,VCH Verlagsgesellschaft mbH, Weinheim, DE; G. KAIBEL et al.: "Möglichkeiten zur Prozessintegration bei destillativen Trennverfahren"

## Beschreibung

Die Erfindung betrifft ein Verfahren zur destillativen Abtrennung geringer Mengen von kleiner 2 Gew.-%, bevorzugt kleiner 0,1 Gew.-%, einer Mittelsiederfraktion aus einem Flüssigkeitsgemisch mittels einer Hauptkolonne, bestehend aus Verstärkungs- und Abtriebsteil, wobei das Flüssigkeitsgemisch größere Mengen an leichter und schwerer siedenden Bestandteilen enthält, und wobei der Verstärkungsteil mit dem oberen Ende und der Abtriebsteil mit dem unteren Ende einer Seitenkolonne verbunden ist, und wobei aus deren mittleren Bereich die Mittelsiederfraktion dampfförmig oder flüssig entnommen wird.

Eine wichtige Grundoperation in der chemischen und der petrochemischen Industrie ist die destillative Auftrennung von Vielkomponentengemischen. Eine Grundregel der Destillationstechnik besagt, daß die Auftrennung eines aus n Komponenten bestehenden Zulaufgemisches in reine Einzelfraktionen n-1 Destillationskolonnen erfordert. Bei Zulaufgemischen, die sich aus zahlreichen Einzelfraktionen zusammensetzen, bedingt dies einen hohen technischen Aufwand.

Störend ist insbesondere, daß dadurch selbst die Entfernung von Verunreinigungen, die nur in geringer Konzentration, beispielsweise in wenigen ppm, im Zulaufgemisch vorliegen, aber aus Qualitätsgründen in den Wertfraktionen abgereichert sein müssen, noch sehr aufwendig ist, da auch solche Abtrennungen eine eigene komplette Kolonneneinheit erfordern. Die Abmessungen einer solchen Destillationsanlage und damit auch die Investitionskosten und der Energieverbrauch liegen in derselben Größenordnung wie bei Kolonneneinheiten zur Entfernung von größeren Produktmengen.

Zur Verringerung des apparativen Aufwands begnügt man sich bei der Entfernung von kleinen Mengen an mittelsiedenden Verunreinigungen üblicherweise mit einer einfachen Seitenentnahme und spart dadurch eine Kolonneneinheit ein. Nachteilig ist hierbei, daß hohe Konzentrationen des Mittelsieders bei einfachen Seitenentnahmen nur bei Anwendung großer Heizleistungen erreichbar sind und so die Wirtschaftlichkeit beeinträchtigen. In der Regel verzichtet man daher auf hohe Mittelsiederkonzentrationen und nimmt Produktverluste an Leichtsieder bzw. Hochsieder in Kauf.

Bei größeren Zulaufkonzentrationen an der mittelsiedenen Komponente können einfache Seitenabzüge aus den genannten Gründen nicht eingesetzt werden. Hier versucht man teilweise, mit Seitenkolonnen zu arbeiten, damit durch diese Maßnahme zumindest ein Wärmetauscher, je nach Kolonnentyp ein Verdampfer oder ein Kondensator, eingespart werden kann. Der Energiebedarf verringert sich jedoch kaum.

Größere Energieeinsparungen und Einsparungen an Investitionskosten von etwa 30 % liefern nur eine Trennwandkolonne oder die thermodynamisch äquivalente Anordnung einer Hauptkolonne mit angeschlossener Seitenkolonne, bei der die Seitenkolonne sowohl an ihrem oberen als auch an ihrem unteren Kolonnenende mit der Hauptkolonne direkt verbunden ist. Diese Kolonnentypen ermöglichen die Auftrennung eines Zulaufgemisches in drei reine Komponenten, wobei nur ein Verdampfer sowie ein Kondensator benötigt werden.

Bei der Trennwandkolonne und der thermodynamisch äquivalenten Anordnung von Haupt- und Seitenkolonne wird dabei allgemein eine Brüdenaufteilung auf Zulauf- und Entnahmeteil der Trennwandkolonne bzw. den entsprechenden als separate Kolonnenschüsse ausgebildeten Teilkolonnen von bevorzugt 1:1 verwirklicht. Dies ist bei einer Trennwandkolonne aus konstruktiven Gründen anzustreben, da sich dabei eine einfachere Geometrie der Kolonneneinbauten ergibt. Auch die theoretischen Untersuchungen befassen sich ausschließlich damit, die Brauchbarkeit dieser gleichmäßigen Brüdenaufteilung auf die beiden Kolonnenteilbereiche nachzuweisen.

Entsprechendes gilt für die konstruktive Alternativausführung in Form von zwei separaten Kolonnen. Auch hier ist eine gleichmäßige Brüdenaufteilung bevorzugt, und es wird lediglich darauf hingewiesen, daß es im allgemeinen nur wenig Mehraufwand an Energie bedeutet, wenn man von der als optimal angesehenen 1:1 Aufteilung ungewollt abweicht. Die Tolerierbarkeit von Schwankungen in der Brüdenaufteilung auf Zulauf- und Entnahmeteil in der maximalen Spanne von 1:3 bis 3:1 wird als Vorteil dieses Kolonnentyps beschrieben. Grundsätzlich wird aber immer die 1:1 Aufteilung als günstigste Lösung angesehen.

Damit verschenkt man unbewußt einen wichtigen Vorteil dieser Kolonnenanordnung. Die Anwendung dieses Kolonnentyps wird damit ebenso starr wie die der herkömmlichen Kolonnen, d.h. jeder einzelne Destillationsschritt eines Verfahrens muß detailliert vorab untersucht werden. Auch die Abtrennung kleiner Mengen von Verunreinigungen muß durch genaue Untersuchungen in Versuchsanlagen vorab geklärt werden und ein entsprechender destillativer Trennschritt konventionell oder mit den beschriebenen modernen Kolonnentypen eigens geplant und ausgeführt werden.

Wie die Praxis zeigt, ist dies oftmals nur schwer möglich. Es ist bei neuen Verfahren häufig sehr schwierig, kleine Verunreinigungen experimentell richtig zu erfassen und ihre apparatetechnischen Konsequenzen vor Bau der Produktionsanlage richtig zu ermitteln. Hieraus folgt beispielsweise die Notwendigkeit einer Vielzahl von Rückführungszyklen bei Versuchsanlagen, was die Versuchsdauer, die Anforderungen an die Analytik und damit die Kosten erhöht. Hinzu kommt, daß ohnehin nicht alle Effekte zu erfassen sind. Neue Nebenprodukte können noch während der Produktionsphase auftreten, wenn beispielsweise ein neuer wirtschaftlicherer Katalysatortyp zur Anwendung kommt, Einsatzstoffe verschiedener Herkunft benutzt werden oder andere verfahrenstechnische Änderungen vorgenommen werden.

Häufig können daher solche verfahrenstechnische und damit wirtschaftliche Verbesserungen nicht zum Tragen kommen, da die zusätzliche Entfernung einer, wenn auch nur in geringen Mengen vorliegenden Komponente einen zusätzlichen zwischengeschalteten Destillationsschritt erforderlich macht und dadurch neben den eigentlichen zusätzlichen Investitionskosten vor allem die in der Regel noch ungleich höheren Stillstandskosten für den Umbau verursacht.

Die US-Patentschrift 4 244 869 beschreibt beispielsweise ein Verfahren zur Reinigung von Rohmethanol mittels einer Rektifikationskolonne, einer vorgeschalteten Kopfkolonne und einer nachgeschalteten Hilfskolonne.

Es stellte sich daher die Aufgabe, geringe Mengen einer Mittelsiederfraktion aus einem Flüssigkeitsgemisch abzutrennen und dabei all die vorab aufgezeigten Nachteile zu vermeiden.

Diese Aufgabe wird erfindungsgemäß dadurch gelöst, daß vom Abtriebsteil der Hauptkolonne Brüden in einer Menge von 1-20 Gew.-%, bevorzugt 3-10 Gew.-%, bezogen auf die Brüdenmenge in der Hauptkolonne an der betreffenden Stelle in das untere Ende der Seitenkolonne geleitet werden.

Weitere Merkmale des erfindungsgemäßen Verfahrens sind Gegenstand der abhängigen Ansprüche.

Entgegen der bisher vertretenen Meinung zeigt es sich, daß in diesem Fall der Abtrennung kleiner Mengen auch eine von der im allgemeinen als ideal erachteten 1:1-Brüdenaufteilung stark abgewichen werden kann, indem man die zusätzliche Seitenkolonne, die zur Entnahme des mittelsiedenen Nebenproduktes dient, mit nur sehr geringen Brüdenmengen von etwa 3-10 % bezogen auf die Brüdenmenge in der Hauptkolonne beaufschlagt. Dies bietet den Vorteil, daß diese Seitenkolonne geringe Investitions- und Energiekosten verursacht.

Die Auftrennung zwischen Leichtsieder- und Schwersiederfraktion in der Hauptkolonne wird durch diese Maßnahme nicht negativ beeinflußt. Da die Konzentration des Mittelsieders in der Hauptkolonne durch die angeschlossene Seitenkolonne verringert wird, ergeben sich sogar geringfügig günstigere Trennleistungen.

Als besonderer Vorteil ist zu sehen, daß bei der erfindungsgemäßen Entfernung von kleinen Mengen an Mittelsieder über eine im Durchmesser im Verhältnis zur Hauptkolonne sehr kleine Seitenkolonne mit der Mittelsiederfraktion nur kleine Mengen von der Leicht- und Schwersiederfraktion verloren gehen, wenn man eine einfache Seitenentnahme als Vergleichsfall gegenüberstellt.

Hinsichtlich der Trennstufenzahl der Seitenkolonne bestehen keine besonderen Anforderungen, d.h. sie kann die gleiche, eine höhere oder niedrigere theoretische Trennstufenzahl, verglichen mit dem entsprechenden Abschnitt der Hauptkolonne, aufweisen. Auch der Typ der Trenneinbauten ist unabhängig von der Hauptkolonne. Da die Seitenkolonne nur eine kleine Querschnittsfläche und damit geringe Kosten aufweist, wird man sie meist etwas aufwendiger hinsichtlich der theoretischen Trennstufenzahl ausstatten und diese, verglichen mit der Hauptkolonne, um etwa 20-50 % erhöhen.

Die genaue Lage der Anschlußstutzen an der Hauptkolonne im Verstärkungs- und Abtriebsteil richtet sich nach den im Einzelfall vorliegenden Trennspezifikationen und kann, wenn die Siedeeigenschaften der verunreinigenden Mittelsiederkomponente bekannt sind, gegebenenfalls durch eine rechnerische Simulation bestimmt werden. Häufig sind diese Stoffeigenschaften jedoch nicht bekannt. In diesem Fall wählt man bei schärferen Reinheitsanforderungen die Lage der Verbindungsstellen zwischen Haupt- und Seitenkolonne so, daß sie näher an der Zulaufstelle der Hauptkolonne liegen. In vielen Fällen erweist es sich als zweckmäßig, die Verbindungsstellen in der Mitte des Verstärkungs- und des Abtriebsteils der Hauptkolonne anzuordnen.

Die Anordnung der Seitenentnahmestelle an der Seitenkolonne sollte sich nach der Lage der Zulaufstelle in der Hauptkolonne bezüglich der Anschlußstellen richten. Bei mittiger Anordnung an der Hauptkolonne empfiehlt es sich, die Seitenentnahmestelle in der Seitenkolonne ebenfalls in der Mitte anzuordnen. Liegt jedoch die Zulaufstelle näher an der oberen Verbindungsstelle, so sollte die Entnahmestelle näher an der unteren Verbindungsstelle liegen und umgekehrt.

### Beispiel (vgl. Zeichnung)

Ein Zulaufgemisch 1 bestehend aus 16.263 kg/h Cyclohexanon, 20.676,5 kg/h Cyclohexanol, 6,4 kg/h Methylcyclohexanon und 1.741,6 kg/h höher siedende Nebenprodukte wird flüssig mit einer Temperatur von 110°C auf dem zehnten theoretischen Boden einer Destillationskolonne mit insgesamt 48 theoretischen Trennstufen eingespeist. Der Betriebsdruck dieser Kolonne beträgt am Kopf 40 mbar und am Sumpf 64 mbar. Am Kopf 2 dieser Kolonne wird bei einer Temperatur von 62,3°C und einem Rücklaufverhältnis von 2,9 Cyclohexanon in einer Menge von 15.117 kg/h entnommen. Das Kopfprodukt enthält noch 30 ppm Methylcyclohexanon. Am Sumpf 3 werden bei 88,7°C 1.128,7 kg/h Cyclohexanon, 20.671,3 kg/h Cyclohexanol, 4,25 kg/h Methylcyclohexanon und 1.741,6 kg/h Hochsieder entnommen.

Zwischen dem untersten Kolonnenboden und dem 29. theoretischen Boden ist eine Entnahmekolonne mit insgesamt 35 theoretischen Trennstufen angeschlossen. Am oberen Ende werden bei einer Temperatur von 66,8°C etwa 4.500 kg/h Flüssigkeit aus der Hauptkolonne aufgegeben und am unteren Ende etwa 4.300 kg/h Brüden aus der Hauptkolonne eingespeist. In Höhe des 10. theoretischen Bodens dieser Kolonne 4 werden flüssig bei 74°C 25 kg/h Mittelsiederfraktion entnommen, die 72,2 % Cyclohexanon, 21 % Cyclohexanol und 6,8 % Methylcyclohexanon enthalten.

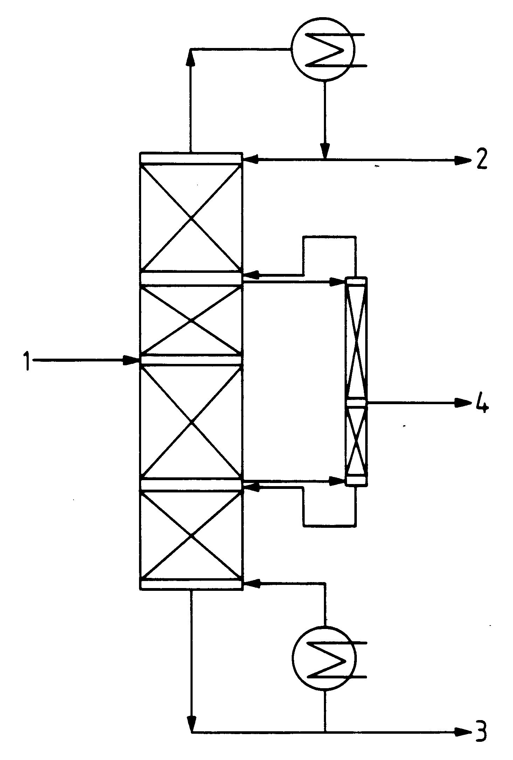

## Patentansprüche

1. Verfahren zur destillativen Abtrennung geringer Mengen von kleiner 2 Gew.-%, bevorzugt kleiner 0,1 Gew.-%, einer Mittelsiederfraktion aus einem Flüssigkeitsgemisch mittels einer Hauptkolonne, bestehend aus Verstärkungs- und Abtriebsteil, wobei das Flüssigkeitsgemisch größere Mengen an leichter und schwerer siedenden Bestandteilen enthält, und wobei der Verstärkungsteil mit dem oberen Ende und der Abtriebsteil mit dem unteren Ende einer Seitenkolonne verbunden ist, und wobei aus deren mittleren Bereich die Mittelsiederfraktion dampfförmig oder flüssig entnommen wird, dadurch gekennzeichnet, daß vom Abtriebsteil der Hauptkolonne Brüden in einer Menge von 1-20 Gew.-%, bevorzugt 3-10 Gew.-%, bezogen auf die Brüdenmenge in der Hauptkolonne an der betreffenden Stelle in das untere Ende der Seitenkolonne geleitet werden.

2. Verfahren nach Anspruch 1, dadurch gekennzeichnet, daß die Entnahmestelle der Seitenkolonne so angeordnet wird, daß die theoretische Trennstufenzahl des Kolonnenabschnitts der Hauptkolonne zwischen Zulaufstelle und oberer Verbindungsstelle zu dem zwischen Zulaufstelle und der unteren Verbindungsstelle gleich dem Verhältnis der theoretischen Trennstufenzahlen der Kolonnenabschnitte der Seitenkolonne unterhalb der Entnahmestelle zu denen oberhalb der Entnahmestelle ist.

3. Verfahren nach den Ansprüchen 1 bis 2, dadurch gekennzeichnet, daß die theoretische Trennstufenzahl der Seitenkolonne, verglichen mit dem entsprechenden Abschnitt der Hauptkolonne zwischen den Anschlußstutzen, 100-200 %, bevorzugt 120-140 %, beträgt.

4. Verfahren nach den Ansprüchen 1 bis 3, dadurch gekennzeichnet, daß am oberen Ende der Seitenkolonne Flüssigkeit aufgegeben wird, die aus der Hauptkolonne entnommen wird, wobei diese Flüssigkeitsmenge 100-110 Gew.-% der Brüdenmenge in der Seitenkolonne entspricht.

5. Verfahren nach den Ansprüchen 1 bis 4, dadurch gekennzeichnet, daß die Mittelsiederfraktion flüssig entnommen wird und an der Entnahmestelle der Seitenfraktion in der Seitenkolonne eine Flüssigkeitsmenge entsprechend einer Verweilzeit von 0,1 bis 2 Stunden in der Kolonne oder in einem separaten Behältnis gehalten wird und die Seitenentnahme nur taktweise nach entsprechender Analyse der Flüssigkeit an der Entnahmestelle erfolgt.

6. Verfahren nach den Ansprüchen 1 bis 5, dadurch gekennzeichnet, daß das Zulaufgemisch an Leichtsiederanteilen 40 bis 65 Gew.% Cyclohexanon, an Mittelsiederanteilen 0,001 bis 0,5 Gew.% Methylcyclohexanon, an Hochsiederanteilen 30 bis 60 Gew.% Cyclohexanol und 1 bis 10 Gew.% höhersiedende Nebenprodukte enthält, die Kolonne bei einem Kopfdruck von 0,01 bis 1 bar, bevorzugt 0,03 bis 0,06 bar, und bei einem Rücklaufverhältnis von 1,2 bis 6,0, bevorzugt 1,8 bis 3,0, betrieben wird, die theoretische Trennstufenzahl der Hauptkolonne 30 bis 65, bevorzugt 40 bis 50, beträgt, die Seitenkolonne eine theoretische Trennstufenzahl von 30 bis 80, bevorzugt von 30 bis 40, besitzt, am Kolonnenkopf der Hauptkolonne eine Cyclohexanonfraktion mit einem Gehalt von 0,0001 bis 0,02 Gew.% Methylcyclohexanon, am Kolonnensumpf der Hauptkolonne eine Cyclohexanolfraktion mit einem Gehalt von 0,002 bis 0,4 Gew.% Methylcyclohexanon und an der Entnahmestelle der Seitenkolonne eine Mittelsiederfraktion mit 1 bis 25 Gew.% Methylcyclohexanon entnommen wird.

## Claims

1. A process for removing small amounts of less than 2, preferably less than 0.1, % by weight of a medium-boiling fraction from a liquid mixture by distillation using a main column consisting of a rectifying part and a stripping part, the liquid mixture containing relatively large amounts of lower-boiling and higher-boiling components, and the rectifying part being connected to the upper end of a side column and the stripping part being connected to the lower end of this column, and the medium-boiling fraction being removed in vapor or liquid form from its middle section, which comprises passing vapors from the stripping part of the main column into the lower end of the side column in an amount of 1-20, preferably 3-10, % by weight, based on the amount of vapor in the main column at the relevant point.

2. A process as claimed in claim 1, wherein the withdrawal point of the side column is arranged in such a way that the ratio of the number of theoretical plates of the section of the main column between the feed point and the upper connection point to that between the feed point and the lower connection point is equal to the ratio of the numbers of theoretical plates of the sections of the side column below the withdrawal point to those above the withdrawal point.

3. A process as claimed in claim 1 or 2, wherein the number of theoretical plates of the side column is 100-200%, preferably 120-140%, compared with the corresponding section of the main column between the connections.

4. A process as claimed in any of claims 1 to 3, wherein liquid removed from the main column is introduced at the upper end of the side column, this amount of liquid corresponding to 100-110% by weight of the amount of vapor in the side column.

5. A process as claimed in any of claims 1 to 4, wherein the medium-boiling fraction is removed in the form of a liquid and, at the withdrawal point of the side fraction in the side column, an amount of liquid is kept for a residence time of from 0.1 to 2 hours in the column or in a separate container, and a side stream is removed at the withdrawal point only periodically after appropriate analysis of the liquid.

6. A process as claimed in any of claims 1 to 5, wherein the feed mixture contains from 40 to 65% by weight of cyclohexanone as the low-boiling fraction, from 0.001 to 0.5% by weight of methylcyclohexanone as the medium-boiling fraction and from 30 to 60% by weight of cyclohexanol and from 1 to 10% by weight of higher-boiling byproducts as the high-boiling fraction, the column is operated at a top pressure of from 0.01 to 1, preferably from 0.03 to 0.06, bar and with a reflux ratio of from 1.2 to 6.0, preferably from 1.8 to 3.0, the number of theoretical plates of the main column is from 30 to 65, preferably from 40 to 50, the side column has from 30 to 80, preferably from 30 to 40, theoretical plates, a cyclohexanone fraction containing from 0.0001 to 0.02% by weight of methylcyclohexanone is removed at the top of the main column, a cyclohexanol fraction containing from 0.002 to 0.4% by weight of methylcyclohexanone is removed at the bottom of the main column and a medium-boiling fraction containing from 1 to 25% by weight of methylcyclohexanone is removed at the withdrawal point of the side column.

## Revendications

1. Procédé pour séparer par distillation de faibles proportions, inférieures à 2% en poids et de préférence inférieures à 0,1% en poids, d'une fraction d'ébullition moyenne d'avec un mélange liquide, à l'aide d'une colonne principale, qui se compose d'une partie de concentration et d'une partie d'épuisement, où le mélange liquide contient de plus fortes proportions de consituants à points d'ébullition moins élevés et plus élevés et où la partie de concentration est raccordée à l'extrémité supérieure et la partie d'épuisement est raccordée à l'extrémité inférieure d'une colonne latérale et où à partir de sa zone médiane on prélève la fraction d'ébullition moyenne sous forme de vapeur ou sous forme liquide, caractérisé en ce que, de la partie épuisement de la colonne principale, on envoie des vapeurs en une proportion de 1 à 20% en poids, de préférence 3 à 10% en poids, par rapport à la proportion de vapeur dans la colonne principale, à l'endroit concerné dans l'extrémité inférieure de la colonne latérale.

2. Procédé suivant la revendication 1, caractérisé en ce que l'endroit de prélèvement de la colonne latérale est agencé en une manière telle que le nombre théorique d'étages de séparation de la section de colonne de la colonne principale entre l'endroit d'addition ou d'alimentation et l'endroit de raccordement supérieur par rapport à la section de colonne entre l'endroit d'addition ou d'alimentation et l'endroit de raccordement inférieur soit égal au rapport des nombres théoriques d'étages de séparation des sections de colonne de la colonne latérale en desous de l'endroit de prélèvement par rapport à ceux au-dessus de l'endroit de prélèvement.

3. Procédé suivant les revendications 1 et 2, caractétion de la colonne latérale, comparé à la section correspondante de la colonne principale entre les tubulures de raccordement varie de 100 à 200%, de préférence 120 à 140%.

4. Procédé suivant les revendications 1 à 3, caractérisé en ce qu'à l'extrémité supérieure de la colonne latérale on introduit du liquide prélevé de la colonne principale, où cette proportion de liquide correspond à 100-110% en poids de la proportion de vapeurs dans la colonne latérale.

5. Procédé suivant les revendications 1 à 4, caractérisé en ce que l'on prélève la fraction à ébullition moyenne à l'état liquide et, à l'endroit de prélèvement de la fraction latérale dans la colonne latérale, on maintient une proportion de liquide correspondant à une durée de séjour de 0,1 à 2 heures dans la colonne ou dans un récipient séparé et le prélèvement latéral ne s'effectue seulement que de manière intermittente après analyse correspondante du liquide à l'endroit de prélèvement.

6. Procédé suivant les revendications 1 à 5, caractérisé en ce que le mélange d'addition ou d'alimentation contient en fractions à basse ébullition 40 à 65% en poids de cyclohexanone, en fractions à ébullition moyenne 0,001 à 0,5% en poids de méthylcyclohexanone, en fractions à ébullition élevée 30 à 60% en poids de cyclohexanol et 1 à 10% en poids de produits secondaires à ébullition plus élevée encore, on fait servir la colonne à une pression de tête de 0,01 à 1 bar, de préférence 0,03 à 0,06 bar et avec un rapport de reflux de 1,2 à 6,0, de préférence 1,8 à 3,0, le nombre théorique d'étages de séparation de la colonne principale varie de 30 à 65, de préférence 40 à 50, la colonne latérale possède un nombre théorique d'étages de séparation de 30 à 80, de préférence 30 à 40, en tête de colonne de la colonne principale on prélève une fraction de cyclohexanone d'une teneur en méthylcyclohexanone de 0,0001 à 0,02% en poids, au fond de colonne de la colonne principale, on prélève une fraction de cyclohexanol d'une teneur en méthylcyclohexanone de 0,002 à 0,4% en poids et à l'endroit de prélèvement de la colonne latérale, on prélève une fraction à ébullition moyenne comportant de 1 à 25% en poids de méthylcyclohexanone.
